# EUROPEAN PATENT APPLICATION

(11) **EP 3 476 823 A1**
(43) Date of publication of application: **01.05.2019**
(21) Application number: 17815315.1
(22) Date of filing: 16.06.2017
(51) Int. Cl.: C07C 49/92, C07F 15/00

(54) **IRIDIUM COMPOUND AND METHOD FOR PRODUCING IRIDIUM COMPLEX BY USING IRIDIUM COMPOUND**

(30) Priority: 24.06.2016 JP 2016125398
(71) Applicant: National Institute of Advanced Industrial Science and Technology, Tokyo 100-8921 (JP); Tanaka Kikinzoku Kogyo K.K., Chiyoda-ku Tokyo 100-6422 (JP)
(72) Inventor: KONNO, Hideo, Tsukuba-shi Ibaraki 305-8565 (JP); TANIUCHI, Junichi, Tsukuba-shi Ibaraki 300-4247 (JP); KOBAYASHI, Rumi, Tsukuba-shi Ibaraki 300-4247 (JP); MASAHIRO, Yasushi, Tokyo 100-6422 (JP)
(74) Representative: Hoffmann Eitle
(86) International application number: PCT/JP2017/022403
(87) International publication number: WO 2017/221847

(57) **Abstract**

The present invention provides an iridium compound useful as a raw material compound for producing a cyclometalated iridium complex, the iridium compound being represented by the following Genera! Formula (1). The iridium compound of the present invention is particularly useful as a raw material for imidazole-based cyclometalated iridium complexes among cyclometalated iridium complexes.

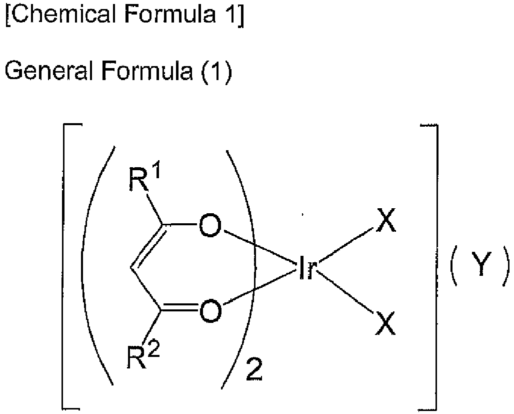

(In General Formula (1), Ir represents an iridium atom, O represents an oxygen atom, X represents a halogen atom, and Y represents a counter cation; and R¹ and R² each independently represent an alkyl group with a carbon number of 1 or more and 10 or less, with the proviso that only one of R¹ and R² is a branched alkyl group.)

## Description

### Technical Field

The present invention relates to a novel iridium compound as a starting material for a phosphorescent material which is used for organic electroluminescent (EL) devices, organic electrochemiluminescent (ECL) devices, luminescent sensors, photosensitizing pigments, photocatalysts, luminescent probes, various light sources and the like. The present invention also relates to a method for producing an iridium complex by use of the iridium compound.

### Background Art

Organic EL devices obtained by use of a phosphorescent material are known to have light-emitting efficiency 3 to 4 times higher than that of organic EL devices obtained by use of a conventional fluorescent material. Thus, the phosphorescent material is essential to achieve higher efficiency and energy saving in organic EL devices. It is a cyclometalated iridium complex that is known as a compound which is used for the phosphorescent material. "Cyclometalated iridium complex" is a general term for organic iridium complexes, in which multidentate ligands are coordinated to the iridium atom to form a ring, and at least one iridium-carbon bond is present.

As a specific example of the cyclometalated iridium complex useful for the phosphorescent material, a cyclometalated iridium complex in which a 2-phenylpyridine derivative is coordinated to iridium as shown in Chemical Formula 1 is well known (tris(2-phenylpyridinato)iridium (Ir(ppy)₃): Patent Document 1). A cyclometalated iridium complex in which a 2-phenylimidazole derivative is coordinated to iridium as shown in Chemical Formula 2 is also known (Patent Document 2: hereinafter, the cyclometalated iridium complex in which a 2-phenylimidazole derivative is coordinated is sometimes referred to as an imidazole-based cyclometalated iridium complex). These cyclometalated iridium complexes can be produced by reacting a raw material composed of an iridium compound with a ligand.

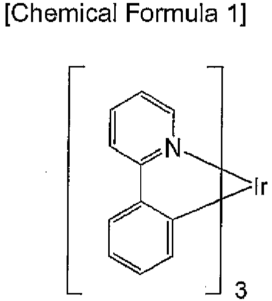

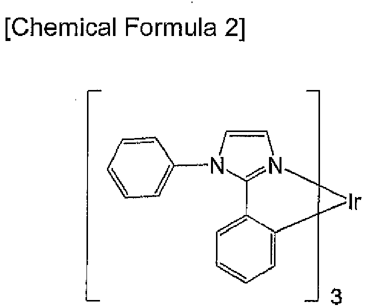

Here, as an iridium compound that forms a raw material for producing a cyclometalated iridium complex, an acetylacetonate compound of iridium has been heretofore well known. For example, Patent Document 1 discloses that by reacting bis(acetylacetonate)dichloroiridium (III) acid sodium with 2-phenylpyridine, the cyclometalated iridium complex of Chemical Formula 1 can be obtained with a high yield. In addition, Patent Document 2 discloses a method in which trisacetylacetonate iridium (III) is reacted with a 2-phenylimidazole derivative ligand to produce the imidazole-based cyclometalated iridium complex of Chemical Formula 2.

### Related Art Document

### Patent Documents

Patent Document 1: WO 2004/085449 A1
Patent Document 2: WO 2006/046980 A1

### Summary of the Invention

### Problems to be Solved by the Invention

However, studies conducted by the present inventors have revealed that when the bis(acetylacetonate)dichloroiridium (III) acid sodium in Patent Document 1 is used, the cyclometalated iridium complex of Chemical Formula 1 can be produced with a favorable yield, but the yield is significantly reduced in production of the imidazole-based cyclometalated iridium complex of Chemical Formula 2.

In addition, the trisacetylacetonate iridium (III) in Patent Document 2 has the problem that this iridium compound has poor reactivity, and requires a very high reaction temperature in production of the complex (generally 200°C or higher). Further, from this iridium compound, the imidazole-based cyclometalated iridium complex of Chemical Formula 2 can be produced, but the yield of the complex is as low as about 35%.

Thus, a previously known acetylacetonate compound of iridium does not necessarily suitably act as a raw material compound depending on the type of cyclometalated iridium complex to be produced.

The present invention has been made in view of the above background, and provides a suitable iridium compound as a starting material for producing a cyclometalated iridium complex to be used as a phosphorescent material for an organic EL device or the like. In particular, the present invention provides a novel iridium compound with which an imidazole-based cyclometalated iridium complex, which has been heretofore difficult to produce with a high yield from a conventional iridium compound, can be produced with a high yield.

### Means for Solving the Problems

For solving the problems described above, the present inventors extensively conducted studies on synthesis of an imidazole-based cyclometalated iridium complex, specifically on a structure of an iridium compound to which an imidazole-based ligand can be coordinated more favorably than ever before. As a result, the present inventors developed, as a starting material, an iridium compound having a specific structure of the following General Formula (1) in which an asymmetric β-diketone ligand is introduced into iridium. According to the present inventors, use of the novel iridium compound considerably improves the yield of an imidazole-based cyclometalated iridium complex as compared to use of an acetylacetonate-based compound which is a conventional raw material.

The present invention provides an iridium compound which is represented by the following General Formula (1).

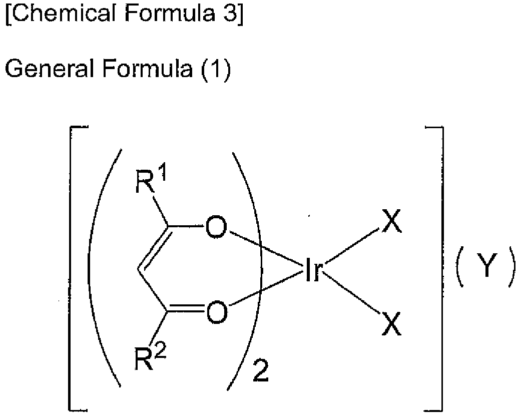

(In General Formula (1), Ir represents an iridium atom, O represents an oxygen atom, X represents a halogen atom, and Y represents a counter cation; and R¹ and R² each independently represent an alkyl group with a carbon number of 1 or more and 10 or less, with the proviso that only one of R¹ and R² is a branched alkyl group.)

Hereinafter, the iridium compound according to the present invention will be described in detail.

As described above, the iridium compound according to the present invention has a structure represented by the following General Formula (1). Here, in General Formula (1), Ir represents an iridium atom, and O represents an oxygen atom.

X represents a halogen atom. Specifically, X is preferably a chlorine atom, a bromine atom or an iodine atom, more preferably a chlorine atom or a bromine atom, especially preferably a chlorine atom.

Y represents a counter cation. The counter cation plays a role in forming a salt by setting the overall charge of the iridium compound of the present invention to 0. Y is not particularly limited as long as it is a cation having the above-mentioned effect. Y is preferably a monovalent cation. Specifically, Y is an alkali metal ion, an ammonium ion, a quaternary ammonium ion, a phosphonium ion, a sulfonium ion, an imidazolium ion, a pyridinium ion, a piperidinium ion, a pyrrolidinium ion, or a proton, preferably an alkali metal ion, an ammonium ion, a quaternary ammonium ion, a phosphonium ion or a sulfonium ion, more preferably an alkali metal ion, especially preferably a sodium ion or a potassium ion, even more preferably a potassium ion.

R¹ and R² each independently represent an alkyl group with a carbon number of 1 or more and 10 or less, and only one of R¹ and R² is a branched alkyl group. In the present invention, the ligand coordinated to the iridium atom is asymmetric while the carbon number of R¹ and R² is limited. This is intended to secure the yield while moderating the conditions (reaction temperature etc.) in synthesis of a cyclometalated iridium complex, particularly an imidazole-based cyclometalated iridium complex by suitably adjusting the stability and reactivity of the iridium compound.

The branched alkyl group that forms one of R¹ and R² in the asymmetric β-diketone ligand is preferably a branched alkyl group with a carbon number of 3 or more and 10 or less, more preferably a branched alkyl group with a carbon number 3 or more and 6 or less. Specifically, the branched alkyl group is preferably an isopropyl group, an isobutyl group, a sec-butyl group, a tert-butyl group or the like, more preferably an isopropyl group.

The alkyl group that forms the other one of R¹ and R² is preferably an alkyl group with a carbon number of 1 or more and 6 or less, more preferably an alkyl group with a carbon number of 1 or more and 3 or less, especially preferably an alkyl group with a carbon number of 1. Specifically, the alkyl group is preferably a methyl group, an ethyl group, a n-propyl group or a n-butyl group, more preferably a methyl group.

The iridium compound according to the present invention has a trans-isomer and a cis-isomer, and when the iridium compound is used as a raw material compound, either the trans-isomer or the cis-isomer may be used. In addition, a mixture of a cis-isomer and a trans-isomer may be used as a raw material.

Further, the iridium compound according to the present invention may have a plurality of geometrical isomers. For example, the compound (Ir-1) of Chemical Formula 4 has geometrical isomers shown in Chemical Formula 5 (indications of counter cations are omitted in Chemical Formula 5). The iridium compound according to the present invention may be formed by only one of these geometrical isomers, or may be a mixture of two or more of these geometrical isomers. In the purpose of producing a cyclometalated iridium complex, whether or not the iridium compound to be used as a raw material is a mixture of geometrical isomers is not particularly important.

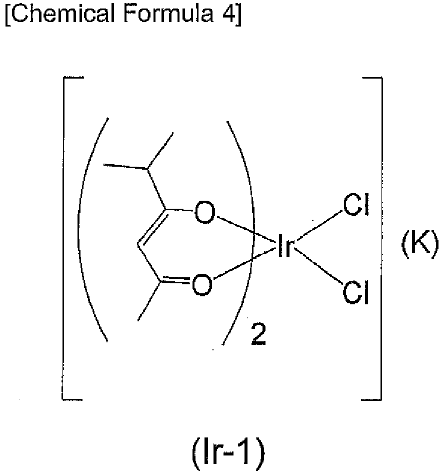

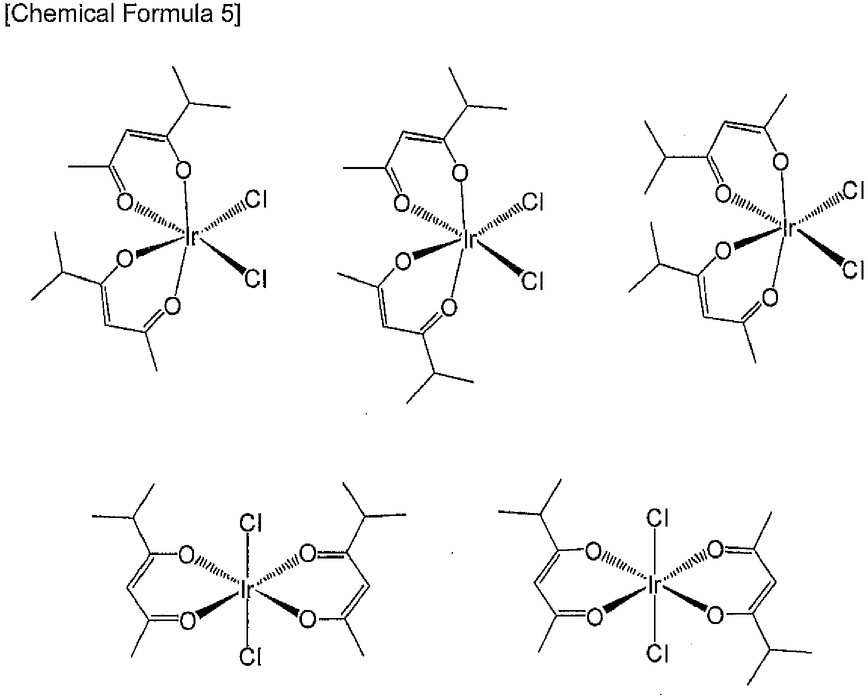

Examples of the iridium compound according to the present invention are shown in Chemical Formula 6, but the iridium compound of the present invention is not limited to these examples.

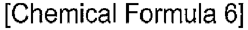

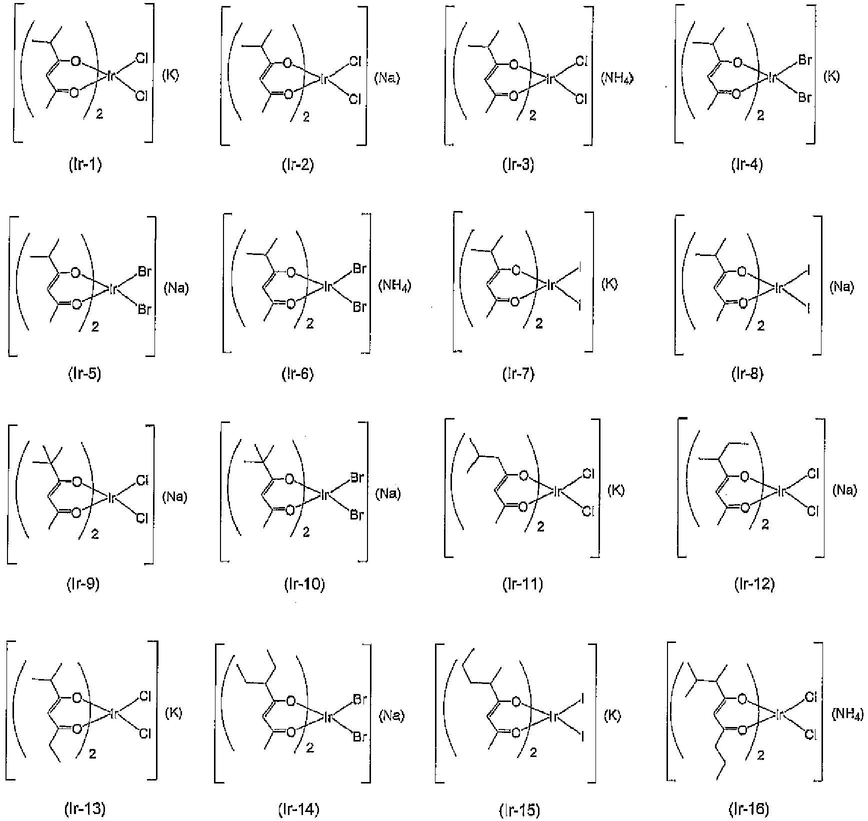

Among the iridium compounds shown in Chemical Formula 6, the iridium compounds (Ir-1) to (Ir-6) are preferable, the iridium compounds (Ir-1) to (Ir-3) are more preferable, and the iridium compound (Ir-1) is especially preferable.

Production of the above-described iridium compound according to the present invention is not difficult, and the iridium compound can be produced by reacting an appropriate iridium compound with a β-diketone ligand having an alkyl group and a branched alkyl group (R¹ and R²). For example, the iridium compound according to the present invention can be synthesized by reacting iridium trichloride n-hydrate with a β-diketone ligand in an aqueous solution containing a hydrogencarbonate (sodium hydrogencarbonate, potassium hydrogencarbonate or the like).

Next, a method for producing a cyclometalated iridium complex by use of the iridium compound according to the present invention will be described. It is not necessary to put a limit on a cyclometalated iridium complex that can be produced by use of the iridium compound according to the present invention. For example, it is possible to produce a cyclometalated iridium complex (Ir(ppy)₃) in which a 2-phenylpyridine derivative is coordinated to iridium as in Chemical Formula 1. In production of the cyclometalated iridium complex, the iridium compound according to the present invention is used as a raw material, and the raw material is reacted with a compound as a ligand.

The iridium compound according to the present invention exhibits its effect particularly in production of an imidazole-based cyclometalated iridium complex in which a 2-phenylimidazole derivative is coordinated to iridium. By using the iridium compound according to the present invention as a raw material, an imidazole-based cyclometalated iridium complex can be produced with a high yield, and such a yield cannot be achieved with a previously known iridium compound.

The method for producing an iridium complex according to the present invention is a method in which the iridium compound according to the present invention and a ligand (2-phenylimidazole derivative) represented by General Formula (2) are reacted with each other to produce an iridium compound of General Formula (3) which is an imidazole-based cyclometalated iridium complex.

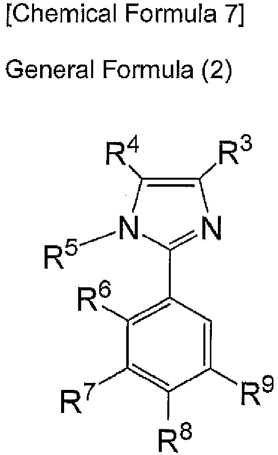

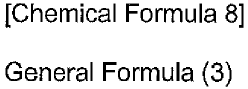

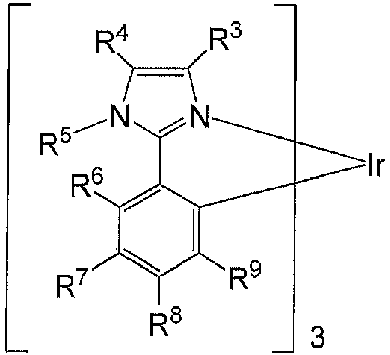

(In General Formulae (2) and (3), Ir represents an iridium atom; N represents a nitrogen atom; R³ to R⁹ each independently represent a hydrogen atom or a substituent; and adjacent substituents may be linked together to further form a ring structure.)

In the 2-phenylimidazole derivative represented by General Formula (2) and the imidazole-based cyclometalated iridium complex represented by Genera! Formula (3), Ir represents an iridium atom, N represents a nitrogen atom, and O represents an oxygen atom. R³ to R⁹ in the general formulae each independently represent a hydrogen atom or a substituent. Adjacent substituents may be linked together to further form a ring structure. Examples of the substituents R³ to R⁹ include the following substituents.
- Alkyl groups (with a carbon number of preferably 1 or more and 30 or less, more preferably 1 or more and 20 or less, especially preferably 1 or more and 10 or less) (e.g., methyl, ethyl, iso-propyl, tert-butyl, n-octyl, n-decyl, n-hexadecyl, cyclopropyl, cyclopentyl and cyclohexyl).
- Alkenyl groups (with a carbon number of preferably 2 or more and 30 or less, more preferably 2 or more and 20 or less, especially preferably 2 or more and 10 or less) (e.g., vinyl, allyl, 2-butenyl and 3-pentenyl).
- Alkynyl groups (with a carbon number of preferably 2 or more and 30 or less, more preferably 2 or more and 20 or less, especially preferably 2 or more and 10 or less) (e.g., propargyl and 3-pentynyl).
- Aryl groups (with a carbon number of preferably 6 or more and 30 or less, more preferably 6 or more and 20 or less, especially preferably 6 or more and 12 or less) (e.g., phenyl, p-methylphenyl, naphthyl and anthranil).
- Amino groups (with a carbon number of preferably 0 or more and 30 or less, more preferably 0 or more and 20 or less, especially preferably 0 or more and 10 or less) (e.g., amino, methylamino, dimethylamino, diethylamino, dibenzylamino, diphenylamino and ditolylamino).
- Alkoxy groups (with a carbon number of preferably 1 or more and 30 or less, more preferably 1 or more and 20 or less, especially preferably 1 or more and 10 or less) (e.g., methoxy, ethoxy, butoxy and 2-ethylhexyloxy).
- Aryloxy groups (with a carbon number of preferably 6 or more and 30 or less, more preferably 6 or more and 20 or less, especially preferably 6 or more and 12 or less) (e.g., phenyloxy, 1-naphthyloxy and 2-naphthyloxy).
- Heterocyclic oxy groups (with a carbon number of preferably 1 or more and 30 or less, more preferably 1 or more and 20 or less, especially preferably 1 or more and 12 or less) (e.g., pyridyloxy, pyrazyloxy, pyrimidyloxy and quinolyloxy).
- Acyl groups (with a carbon number of preferably 1 or more and 30 or less, more preferably 1 or more and 20 or less, especially preferably 1 or more and 12 or less) (e.g., acetyl, benzoyl, formyl and pivaloyl).
- Alkoxycarbonyl groups (with a carbon number of 2 or more and 30 or less, more preferably 2 or more and 20 or less, especially preferably 2 or more and 12 or less) (e.g., methoxycarbonyl and ethoxycarbonyl).
- Aryloxycarbonyl groups (with a carbon number of preferably 7 or more and 30 or less, more preferably 7 or more and 20 or less, especially preferably 7 or more and 12 or less) (e.g., phenyloxycarbonyl).
- Acyloxy groups (with a carbon number of preferably 2 or more and 30 or less, more preferably 2 or more and 20 or less, especially preferably 2 or more and 10 or less) (e.g., acetoxy and benzoyloxy).
- Acylamino groups (with a carbon number of preferably 2 or more and 30 or less, more preferably 2 or more and 20 or less, especially preferably 2 or more and 10 or less) (e.g., acetylamino and benzoylamino).
- Alkoxycarbonylamino groups (with a carbon number of 2 or more and 30 or less, more preferably 2 or more and 20 or less, especially preferably 2 or more and 12 or less) (e.g., methoxycarbonylamino).
- Aryloxycarbonylamino groups (with a carbon number of preferably 7 or more and 30 or less, more preferably 7 or more and 20 or less, especially preferably 7 or more and 12 or less) (e.g., phenyloxycarbonylamino).
- Sulfonylamino groups (with a carbon number of preferably 1 or more and 30 or less, more preferably 1 or more and 20 or less, especially preferably 1 or more and 12 or less) (e.g., methanesulfonylamino and benzenesulfonylamino).
- Sulfamoyl groups (with a carbon number of preferably 0 or more and 30 or less, more preferably 0 or more and 20 or less, especially preferably 0 or more and 12 or less) (e.g., sulfamoyl, methylsulfamoyl, dimethylsulfamoyl and phenylsulfamoyl).
- Carbamoyl groups (with a carbon number of preferably 1 or more and 30 or less, more preferably 1 or more and 20 or less, especially preferably 1 or more and 12 or less) (e.g., carbamoyl, methylcarbamoyl, diethylcarbamoyl and phenylcarbamoyl).
- Alkylthio groups (with a carbon number of preferably 1 or more and 30 or less, more preferably 1 or more and 20 or less, especially preferably 1 or more and 12 or less) (e.g., methylthio and ethylthio).
- Arylthio groups (with a carbon number of preferably 6 or more and 30 or less, more preferably 6 or more and 20 or less, especially preferably 6 or more and 12 or less) (e.g., phenylthio).
- Heterocyclic thio groups (with a carbon number of preferably 1 or more and 30 or less, more preferably 1 or more and 20 or less, especially preferably 1 or more and 12 or less) (e.g., pyridylthio, 2-benzimizolylthio, 2-benzoxazolylthio and 2-benzthiazolylthio).
- Sulfonyl groups (with a carbon number of preferably 1 or more and 30 or less, more preferably 1 or more and 20 or less, especially preferably 1 or more and 12 or less) (e.g., mesyl and tosyl).
- Sulfinyl groups (with a carbon number of preferably 1 or more and 30 or less, more preferably 1 or more and 20 or less, especially preferably 1 or more and 12 or less) (e.g., methanesulfinyl and benzenesulfinyl).
- Ureide groups (with a carbon number of preferably 1 or more and 30 or less, more preferably 1 or more and 20 or less, especially preferably 1 or more and 12 or less) (e.g., ureide, methylureide and phenylureide).
- Phosphoramide groups (with a carbon number of preferably 1 or more and 30 or less, more preferably 1 or more and 20 or less, especially preferably 1 or more and 12 or less) (e.g., diethylphosphoramide and phenylphosphoramide).
- Hydroxyl groups, mercapto groups, halogen atoms (e.g., fluorine atom, chlorine atom, bromine atom and iodine atom), cyano groups, sulfo groups, carboxyl groups, nitro groups, trifluoromethyl groups, hydroxamic acid groups, sulfino groups, hydrazino groups, imino groups and heterocyclic groups (with a carbon number of preferably 1 or more and 30 or less, more preferably 1 or more and 12 or less and having a nitrogen atom, an oxygen atom or a sulfur atom as a heteroatom (specifically, imidazolyl, pyridyl, quinolyl, furyl, thienyl, piperidyl, morpholino, benzoxazolyl, benzimidazolyl, benzothiazolyl, carbazolyl group, azepinyl group and the like).
- Silyl groups (with a carbon number of preferably 3 or more and 40 or less, more preferably 3 or more and 30 or less, especially preferably 3 or more and 24 or less) (e.g., trimethylsilyl and triphenylsilyl).
- Silyloxy groups (with a carbon number of preferably 3 or more and 40 or less, more preferably 3 or more and 30 or less, especially preferably 3 or more and 24 or less) (e.g., trimethylsilyloxy and triphenylsilyloxy).

Among the above-mentioned substituents, alkyl groups, aryl groups, amino groups, alkoxy groups, aryloxy groups, halogen atoms, cyano groups, trifluoromethyl groups, heterocyclic groups and silyl groups are preferable, alkyl groups, aryl groups, halogen atoms, cyano groups and heterocyclic groups are more preferable, and alkyl groups and aryl groups are especially preferable. These substituents may be further substituted with substituents defined by R³ to R⁹.

As a desired form of the aryl group or heterocyclic group, a dendron (a group having a regular dendritic branched structure with a branch point at an atom or ring) is also preferable. Examples of the dendron include structures described in documents such as WO 02/067343 A1, JP 2003-231692 A, WO 2003/079736 A1, WO 2006/097717 A1 and WO 2016/006523 A1.

Desired forms of R³ to R⁹ will be described in further detail.

R³, R⁴, R⁶ and R⁹ are each preferably a hydrogen atom, an alkyl group, a heterocyclic group or an aryl group, more preferably a hydrogen atom, an alkyl group or an aryl group, still more preferably a hydrogen atom.

R⁵ is preferably an alkyl group or an aryl group, more preferably an aryl group, still more preferably a phenyl group, or a phenyl group substituted with an alkyl group.

The method for producing an iridium complex according to the present invention will be described in detail. In the method for producing an iridium complex according to the present invention, it is preferable to use a solvent, in which a reaction proceeds. The solvent is preferably an alcohol, a saturated aliphatic hydrocarbon, an ester, an ether, a nitrile, an aprotic polar solvent, a ketone, an amide, an aromatic hydrocarbon, a nitrogen-containing aromatic compound, an ionic liquid or water. In particular, the solvent is more preferably an alcohol, a saturated aliphatic hydrocarbon, an ester, an ether, an aprotic polar solvent or an amide, especially preferably an alcohol or an aprotic polar solvent (DMF, DMSO or the like), even more preferably an alcohol (with a carbon number of preferably 1 or more and 30 or less, more preferably 1 or more and 20 or less, still more preferably 1 or more and 10 or less). Among alcohols, diol (with a carbon number of preferably 1 or more and 30 or less, more preferably 1 or more and 20 or less, still more preferably 1 or more and 10 or less) is most preferable. Specifically, ethylene glycol, diethylene glycol, triethylene glycol, 1,2-propanediol, 1,3-propanediol or 1,3-butanediol is preferable, with ethylene glycol being more preferable. The above-mentioned solvents may be used singly, or two or more solvents may be used in combination.

In the method for producing an iridium complex according to the present invention, the iridium compound (general formula (1)) as a raw material is dissolved in the solvent, the ligand represented by General Formula (2) is further added, and the mixture is heated and reacted. Here, the concentration of the iridium compound as a raw material in the reaction system is not particularly limited, but is preferably 0.001 mol/L or more and 10.0 mol/L or less, more preferably 0.001 mol/L or more and 1.0 mol/L or less, still more preferably 0.01 mol/L or more and 1.0 mol/L or less, especially preferably 0.05 mol/L or more and 0.5 mol/L or less.

On the other hand, the use amount of the ligand composed of the 2-phenylimidazole derivative represented by General Formula (2) is preferably 2 moles or more and less than 10 moles, more preferably 3 moles or more and less than 10 moles, especially preferably 3 moles or more and less than 8 moles based on 1 mole of the iridium compound as a raw material. When the 2-phenylimidazole derivative is excessively added, removal of the 2-phenylimidazole derivative requires much time. In addition, since the 2-phenylimidazole derivative is expensive, use of an excessive amount of the 2-phenylimidazole derivative increases production cost. Thus, the added amount of the ligand is preferably in the above-mentioned range.

The reaction temperature in the method for producing an iridium complex according to the present invention is more preferably 100°C or higher and lower than 250°C, especially preferably 150°C or higher and lower than 250°C, further especially preferably 150°C or higher and lower than 200°C. In addition, the reaction time is preferably 0.5 hours or more and 72 hours or less, 1 hour or more and 48 hours or less, 1 hour or more and 24 hours or less. The heating means for the reaction system is not particularly limited. Specifically, external heating using an oil bath, a sand bath, a mantle heater, a block heater, or a heat-circulation jacket, as well as heating by irradiation with microwaves can be utilized, for example.

In the method for producing an iridium complex according to the present invention, it is preferable to perform the method in an inert gas (nitrogen, argon or the like) atmosphere. The pressure in the method for producing an iridium complex according to the present invention is not particularly limited, but it is preferable to perform the method under normal pressure (atmospheric pressure).

The iridium complex obtained by the above-described method and represented by General Formula (3) is treated by a general post-treatment method and then, after purification as necessary or without purification, can be used as a high-purity product. As the method for post-treatment, for example, extraction, cooling, crystallization by adding water or an organic solvent, distillation of the solvent from the reaction mixture, and the like may be performed alone or in combination. As the method for purification, recrystallization, distillation, sublimation, column chromatography and the like may be performed alone or in combination.

The iridium complex obtained according to the present invention and represented by General Formula (3) can be suitably used as a phosphorescent material for an organic EL element or the like. Examples of the iridium complex which can be produced according to the present invention and represented by General Formula (3) are shown in Chemical Formula 9.

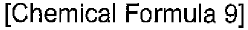

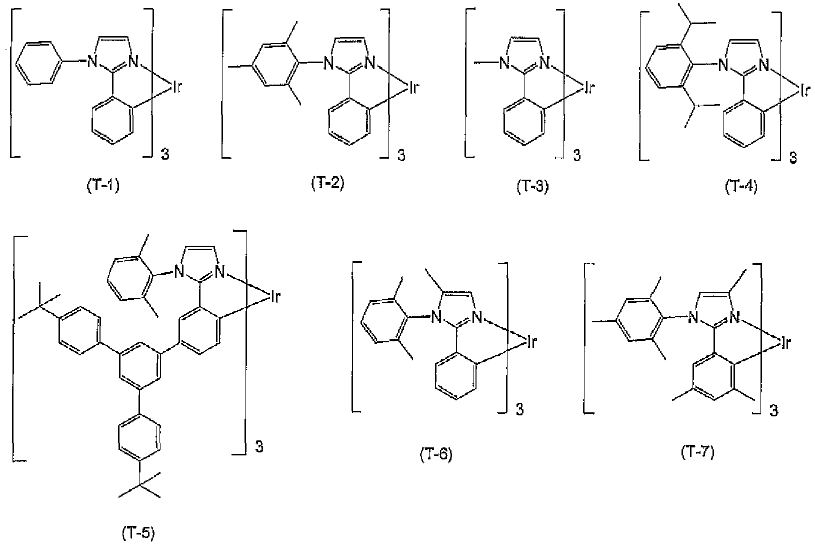

### Advantageous Effects of the Invention

The iridium compound according to the present invention is suitable as a starting material for producing a cyclometalated iridium complex to be used as a phosphorescent material for an organic EL device or the like. Particularly, in the present invention, an imidazole-based cyclometalated iridium complex can also be produced with a high yield.

### Description of Embodiments

Hereinafter, an embodiment of the present invention will be described in detail, but the embodiment is illustrative, and the present invention is not limited thereto.

In this embodiment, the iridium compound (Ir-1) of Chemical Formula 6 was used as a raw material, and reacted with the following 2-phenylimidazole derivative (L-1) to produce an imidazole-based cyclometalated iridium complex (T-1).

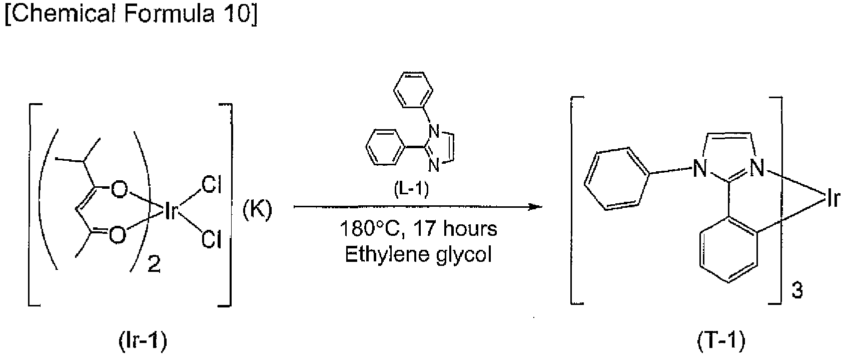

### [Production of iridium compound (Ir-1)]

40.6 g (115 mmol) of iridium trichloride trihydrate and 530 ml of pure water were added in a three-necked flask, and dissolved, 45.7 g (357 mmol) of 5-methyl-2,4-hexanedione was subsequently added, the mixture was reacted at 95°C for 1 hour, and 47.5 g (475 mmol) of potassium carbonate was added to the mixture little by little to adjust the pH to about 8. Further, the mixture was heated and reacted for 5 hours. After the reaction, the reaction product was left standing overnight, unreacted 5-methyl-2,4-hexanedione was extracted and removed from the aqueous layer of the supernatant by use of hexane, an iridium compound (Ir-1) was subsequently extracted with ethyl acetate, and the extract was concentrated and dried to obtain 12 g of an orange crude crystal of the iridium compound (Ir-1). Further, the crude crystal was subjected to column purification to obtain 10.2 g of an orange crystal of the iridium compound (Ir-1). The isolation yield was 16%.

### [Production of iridium complex]

167.0 mg (0.3 mmol) of the iridium compound (Ir-1) produced as described above, 396.0 mg (1.8 mmol) of a 2-phenylimidazole derivative (L-1) and 5 ml of ethylene glycol as a solvent were added in a three-necked flask, and the mixture was heated and reacted in an argon atmosphere at 180°C for 17 hours. After completion of the reaction, the reaction solution was cooled to room temperature, and the precipitated solid was washed with methanol to obtain an imidazole-based cyclometalated iridium complex (T-1). The isolation yield was 73%. The product was analyzed by ¹H-NMR, and identified.

Comparative Example: In the comparative example, bis(acetylacetonate)dichloroiridium (III) acid sodium in Patent Document 1 was used as a raw material, and reacted with the following 2-phenylimidazole derivative (L-1) to produce an imidazole-based cyclometalated iridium complex (T-1).

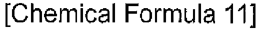

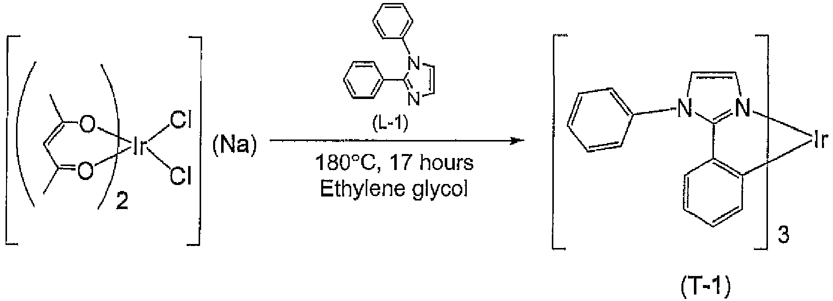

145.3 mg (0.3 mmol) of bis(acetylacetonate)dichloroiridium (III) acid sodium, 396.0 mg (1.8 mmol) of a 2-phenylimidazole derivative (L-1) and 5 ml of ethylene glycol were added in a three-necked flask, and the mixture was heated and reacted in an argon atmosphere at 180°C for 17 hours. After completion of the reaction, the reaction solution was cooled to room temperature, and the precipitated solid was washed with methanol to obtain an imidazole-based cyclometalated iridium complex (T-1). The isolation yield was 52%.

From the above results, it was confirmed that imidazole-based cyclometalated iridium (T-1) was produced with a high yield by use of the iridium compound (Ir-1) of this embodiment. Comparison between this embodiment and the comparative example shows that when the iridium compound (lr-1) of this embodiment was used, the yield was higher by 40% or more than when bis(acetylacetonate)dichloroiridium (III) acid sodium of the comparative example, which is a conventional compound, was used.

### Industrial Applicability

The present invention allows a cyclometalated iridium complex to be efficiently produced. Particularly, an imidazole-based cyclometalated iridium complex can also be produced with a high yield. The present invention provides a raw material suitable for producing a cyclometalated iridium complex to be used as a phosphorescent material which is used for organic electroluminescent (EL) devices, organic electrochemiluminescent (ECL) devices, luminescent sensors, photosensitizing pigments, photocatalysts, luminescent probes, various light sources and the like.

## Claims

1. An iridium compound which is represented by the following General Formula (1): 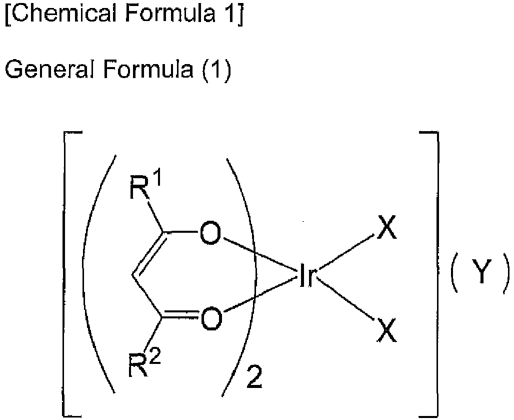 (in General Formula (1), Ir represents an iridium atom, O represents an oxygen atom, X represents a halogen atom, and Y represents a counter cation; and R¹ and R² each independently represent an alkyl group with a carbon number of 1 or more and 10 or less, with the proviso that only one of R¹ and R² is a branched alkyl group).

2. The iridium compound according to claim 1, wherein one of R¹ and R² is a methyl group, and the other one of R¹ and R² is a branched alkyl group with a carbon number of 3 or more and 10 or less.

3. The iridium compound according to claim 1 or 2, wherein one of R¹ and R² is an isopropyl group.

4. The iridium compound according to any one of claims 1 to 3, wherein X represents a chlorine atom or a bromine atom.

5. A method for producing an iridium complex by reacting a raw material including an iridium compound with a ligand, comprising producing an iridium complex by reacting the raw material with the ligand, the raw material being the iridium compound defined in any one of claims 1 to 4, the ligand being a ligand represented by the following General Formula (2), the iridium complex being represented by the following General Formula (3): 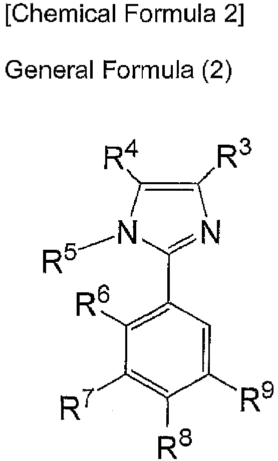 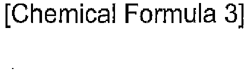 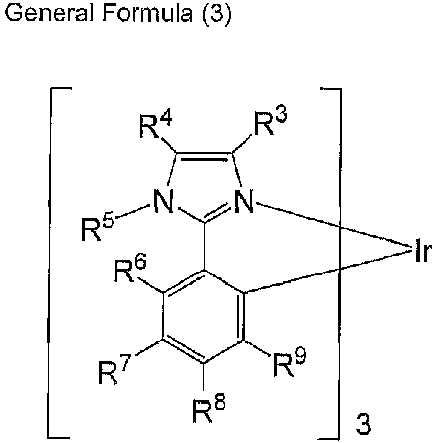 (in General Formulae (2) and (3), Ir represents an iridium atom; N represents a nitrogen atom; R³ to R⁹ each independently represent a hydrogen atom or a substituent; and adjacent substituents are optionally linked together to further form a ring structure).
